(19) Europäisches Patentamt
European Patent Office
Office européen des brevets

(11) **EP 1 427 329 B1**

(12) **FASCICULE DE BREVET EUROPEEN**

(45) Date de publication et mention
de la délivrance du brevet:
**23.11.2011 Bulletin 2011/47**

(21) Numéro de dépôt: **02794809.0**

(22) Date de dépôt: **12.08.2002**

(51) Int Cl.:
***A61B 3/103*** (2006.01)

(86) Numéro de dépôt international:
**PCT/FR2002/002859**

(87) Numéro de publication internationale:
**WO 2003/015622 (27.02.2003 Gazette 2003/09)**

(54) **DISPOSITIF DE MESURE DES ABERRATIONS D'UN SYSTEME DE TYPE OEIL**

VORRICHTUNG ZUR MESSUNG VON ABWEICHUNGEN IN EINEM AUGENARTIGEN SYSTEM

DEVICE FOR MEASURING ABERRATIONS IN AN EYE-TYPE SYSTEM

(84) Etats contractants désignés:
**AT BE BG CH CY CZ DE DK EE ES FI FR GB GR
IE IT LI LU MC NL PT SE SK TR**

(30) Priorité: **12.08.2001 FR 0111112**

(43) Date de publication de la demande:
**16.06.2004 Bulletin 2004/25**

(73) Titulaire: **Imagine Eyes
91400 Orsay (FR)**

(72) Inventeurs:
• **Bucourt, Samuel Henri
91440 Bures sur Yvette (FR)**
• **Levecq, Xavier Jean-François
91110 Gif sur Yvette (FR)**

(74) Mandataire: **Pontet, Bernard
Pontet Allano & Associés s.e.l.a.r.l.
25, Rue Jean Rostand
Parc Club Orsay Université
91893 Orsay Cedex (FR)**

(56) Documents cités:
**US-A- 6 050 687        US-A- 6 086 204
US-B1- 6 264 328**

• **LIANG J ET AL: "OBJECTIVE MEASUREMENT OF WAVE ABERRATIONS OF THE HUMAN EYE WITH THE USE OF A HARTMANN-SHACK WAVE-FRONT SENSOR" JOURNAL OF THE OPTICAL SOCIETY OF AMERICA, AMERICAN INSTITUTE OF PHYSICS. NEW YORK, US, vol. 11, no. 7, juillet 1994 (1994-07), pages 1949-1957, XP000955413 cité dans la demande**

**Description**

L'invention concerne un dispositif de mesure des aberrations d'un système de type oeil, oeil vivant ou oeil artificiel, équipé ou non de verres correcteurs, le résultat de ces mesures permettant notamment de définir avec précision des éléments optiques correctifs de la vision, d'apporter un support à la chirurgie corrective de l'oeil, de détecter des pathologies oculaires, et plus généralement de fournir des mesures précises à tout appareil qui nécessite la connaissance des aberrations de l'oeil. Le dispositif selon l'invention permet également d'établir la topographie de la face antérieure de la cornée.

La plupart des instruments utilisés aujourd'hui par les optométristes pour corriger la vision oculaire ne mesurent que l'amétropie (myopie ou hypermétropie) et l'astigmatisme. Ils ne peuvent pas mesurer l'ensemble des aberrations de l'oeil, notamment la coma, l'aberration sphérique, ou les aberrations d'ordres plus élevés, entraînant une correction approximative de la vision.

Récemment, des dispositifs de mesure des aberrations de l'oeil ont été développés, basés sur l'utilisation d'un analyseur de front d'onde de type Shack-Hartmann (voir par exemple "Objective measurement of wave aberrations of the human eye with the use of a Schack-Hartman wave-front sensor", Liang et al., J.Opt.Soc.Am.A, Vol.11, N°7, pp.1-9, 1994). Dans ce dispositif, l'analyseur de front d'onde permet d'analyser l'onde émergente de l'oeil après focalisation sur la rétine d'un faisceau d'éclairage, générant un point source diffusant. Les performances obtenues sont cependant limitées par les réflexions parasites, sur la cornée et sur les éléments optiques du montage, qui entraînent des imprécisions dans l'analyse du front d'onde. Le montage préconisé par Williams et al. (US 0577719) résout en partie le problème des réflexions parasites par l'utilisation d'une lame séparatrice de polarisation.

Cependant, il est important dans un dispositif de ce type d'optimiser le rendement lumineux entre le flux qui pénètre dans l'oeil et celui qui peut être récupéré pour procéder à l'analyse du front d'onde, afin de limiter, pour le confort du patient, le flux lumineux incident sur la rétine. Or l'utilisation d'une lame séparatrice de polarisation telle qu'elle est préconisée dans le brevet cité entraîne une perte de flux importante sur la voie d'analyse.

Pour remédier notamment à cet inconvénient, l'invention propose un dispositif de mesure des aberrations d'un système de type oeil, comprenant un analyseur de front d'onde, par exemple de type Shack-Hartmann, quai optimise le rendement lumineux entre le flux incident sur la rétine et le flux récupéré pour l'analyse, tout en assurant une très grande fiabilité de la mesure et un montage optique simple.

Pour cela, le dispositif selon l'invention comprend une voie d'éclairage avec notamment des moyens d'émission d'un faisceau d'éclairage pour former par rétrodiffusion sur la rétine de l'oeil une source lumineuse secondaire et une voie d'analyse avec notamment des moyens d'analyse dans un plan d'analyse donné de la phase du front de l'onde émise par ladite source secondaire et émergente de l'oeil, caractérisé en ce qu'il comprend en outre :

- des moyens d'imagerie et des moyens de positionnement de l'oeil permettant notamment le positionnement d'un plan prédéterminé de l'oeil dans un plan focal desdits moyens d'imagerie,

- un élément de filtrage des réflexions parasites dont le centre définit avec le centre optique des moyens d'imagerie un axe de mesure, sensiblement centré sur la pupille de l'oeil,

- des moyens de séparation optique positionnés sur l'axe de mesure définissant la voie d'éclairage et la voie d'analyse,

- sur la voie d'éclairage, un diaphragme d'éclairage d'ouverture prédéterminée,

- des moyens de conjugaison optique centrés sur l'axe de mesure et permettant d'assurer la conjugaison optique entre ledit plan prédéterminé de l'oeil, le plan du diaphragme d'éclairage et le plan d'analyse, et caractérisé en ce que le faisceau d'éclairage est convergent sensiblement au centre de l'élément de filtrage, en ce que l'élément de filtrage, la voie d'éclairage, la voie d'analyse, les moyens de séparation et les moyens de conjugaison sont solidaires, positionnés sur une plate-forme mobile par rapport aux moyens d'imagerie le long de l'axe optique desdits moyens, permettant le réglage, en fonction de l'amétropie de l'oeil, de la conjugaison optique par lesdits moyens de la rétine de l'oeil avec le centre de l'élément de filtrage, et en ce que le diaphragme d'éclairage est décentré par rapport à l'axe de mesure, de telle sorte que le flux lumineux parasite réfléchi par la cornée de l'oeil, les moyens d'imagerie, et tout dioptre situé entre l'élément de filtrage et la cornée, soit détourné de la voie d'analyse par l'élément de filtrage.

D'autres avantages et caractéristiques de l'invention apparaîtront plus clairement à la lecture de la description qui suit, illustrée par les figures qui représentent :

- les figures 1A et 1B, des exemples de montage illustrant le dispositif selon l'invention selon une première variante ;

- la figure 2, un exemple de montage illustrant le dispositif selon l'invention selon une seconde variante ;

- la figure 3, un schéma illustrant un exemple de réalisation des moyens de positionnement de l'oeil dans le dispositif selon l'invention ;

- la figure 4, un schéma illustrant une aide au positionnement latéral de l'oeil ;

- la figure 5, la mise en évidence des réflexions parasites dans le montage décrit figure 1A ;

- la figure 6, la mise en évidence des réflexions parasites dans le cas d'un patient porteur de verres correcteurs ;

- la figure 7, un schéma montrant l'application du dispositif selon l'invention à la topographie cornéenne ;
- la figure 8, un schéma montrant une variante de réalisation du dispositif pour l'application à la mesure de la topographie cornéenne.

Dans ces figures, les éléments identiques sont repérés par les mêmes références.

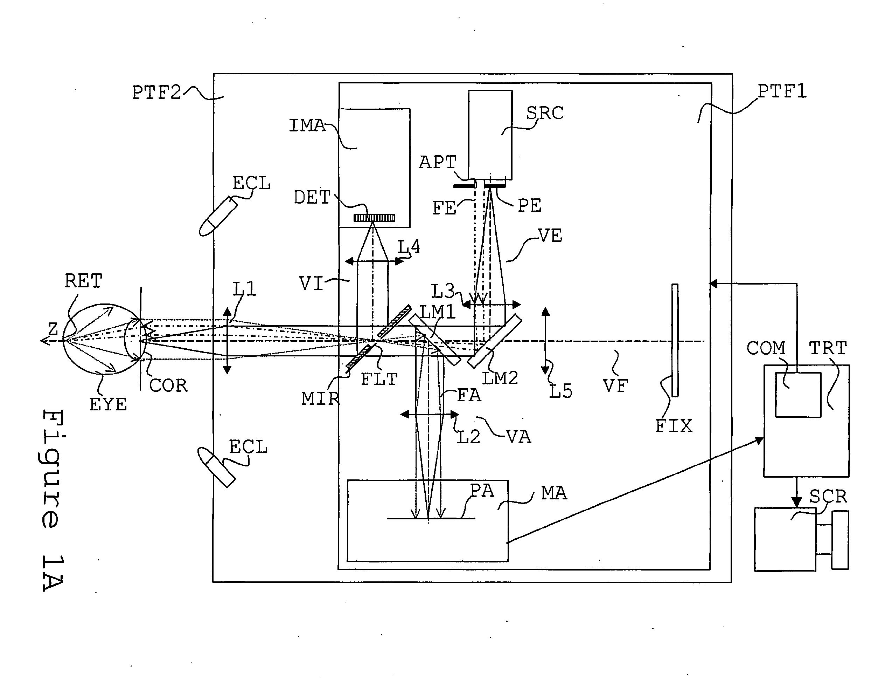

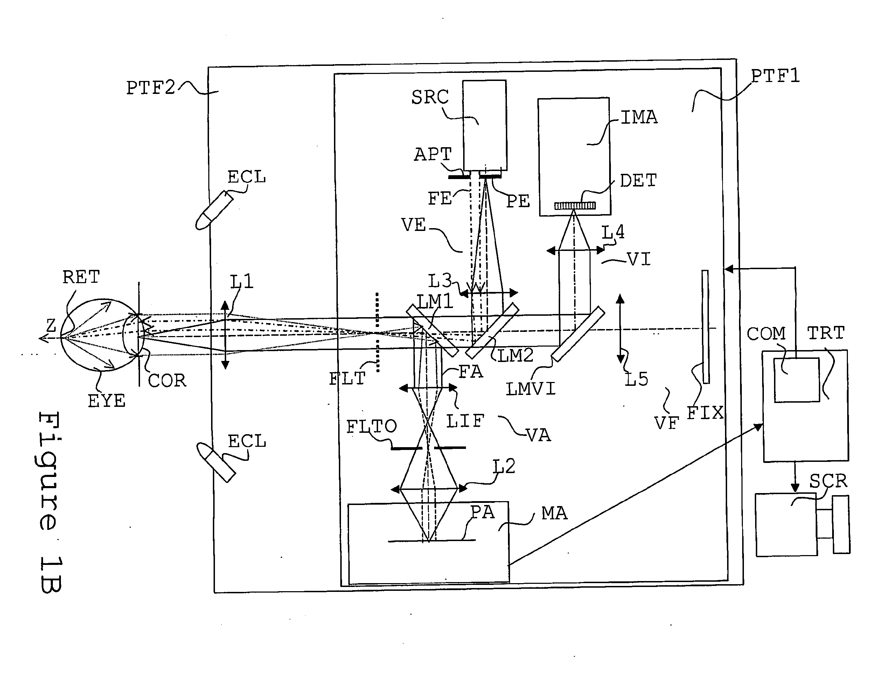

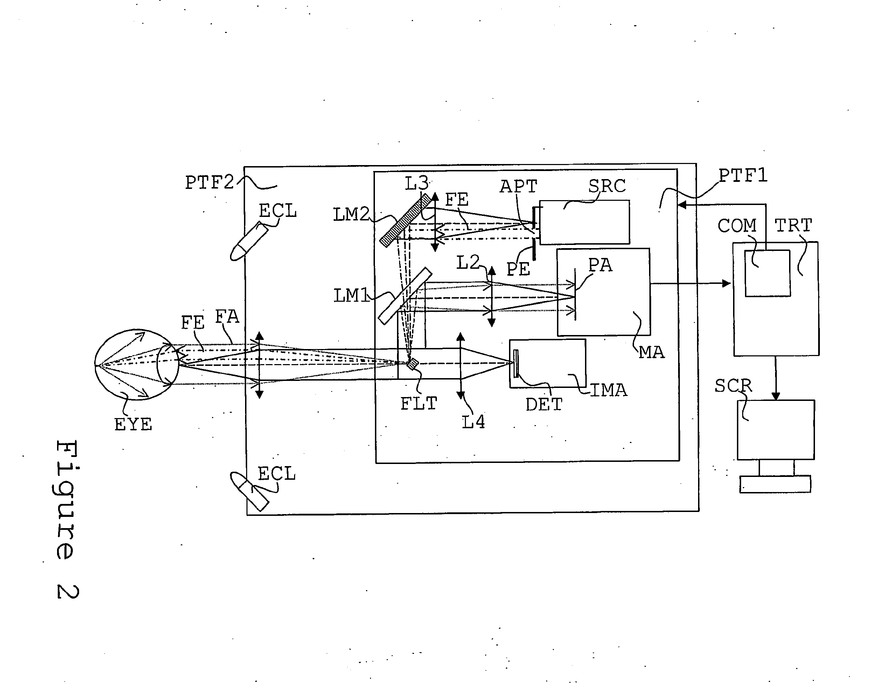

Les figures 1A, 1B et 2 représentent des exemples de montage illustrant le dispositif de mesure des aberrations d'un système de type oeil selon l'invention.

Le dispositif comprend classiquement une voie d'éclairage VE avec notamment des moyens d'émission SRC d'un faisceau d'éclairage FE (en pointillé alterné sur la figure 1A et 1B) pour former sur la rétine RET de l'oeil à analyser EYE un point source lumineux diffusant. Il comprend en outre une voie d'analyse VA avec notamment des moyens d'analyse optique MA dans un plan d'analyse PA donné de la phase du front de l'onde émise par ledit point source et émergente de l'oeil (faisceau FA représenté en pointillé sur la figure 1A et 1B). Les moyens d'analyse, par exemple un analyseur de type Shack-Hartmann, sont reliés à des moyens de traitement TRT, qui, de manière connue, établissent la cartographie en phase de l'onde émergente de l'oeil et calculent les aberrations. Une représentation de cette cartographie peut être affichée sur un écran SCR. Le fonctionnement de l'analyseur Shack-Hartmann, connu de l'art antérieur (voir par exemple l'article « wave-front estimation from wave-front slope measurements », J. Opt. Soc. Am. Vol. 70, No. 8, August 1980), n'est pas repris en détail ici. De façon simplifiée, il comprend habituellement une matrice de microlentilles, définissant le plan d'analyse PA, et un détecteur matriciel. Chacune des microlentilles forme sur le détecteur un point image de la partie du front de l'onde qu'elle intercepte. Les aberrations de l'onde émergente de l'oeil (faisceau FA) entraînent un déplacement des points image sur le détecteur d'une distance proportionnelle à la pente locale du front d'onde. Le signal issu du détecteur est envoyé vers les moyens de traitement TRT qui établissent la cartographie en phase du front de l'onde, permettant d'en déduire les aberrations de l'oeil analysé. Il est à noter cependant que l'invention n'est pas limitée à l'utilisation du Shack-Hartmann et que d'autres analyseurs de front d'onde permettant de déterminer la cartographie en phase d'un front d'onde peuvent être utilisés.

Une des difficultés rencontrées dans un tel dispositif provient de l'élimination des réflexions parasites sur les différents dioptres du montage. En effet, le flux diffusé par la rétine et émergent de l'oeil est très faible et des réflexions parasites intempestives entraînent généralement une saturation de l'image qui rendent l'analyse impossible. Par ailleurs, il est important d'optimiser le rendement lumineux entre le flux incident sur la rétine et le flux émergent de l'oeil après diffusion sur la rétine afin de réduire la quantité de flux lumineux qui pénètre dans l'oeil du patient. Le dispositif mis en oeuvre par les déposants et dont des exemples de réalisation sont représentés sur les figures 1A, 1B et 2, concilie ces contraintes tout en présentant un montage simple et offrant une très bonne fiabilité de mesure.

Pour cela, le dispositif selon l'invention comprend des moyens d'imagerie, par exemple une lentille L1, et des moyens de positionnement de l'oeil permettant notamment le positionnement d'un plan prédéterminé de l'oeil, par exemple le plan de la pupille de l'oeil PO, ou le plan tangent au sommet de la cornée dans un plan focal de la lentille L1. Il comprend en outre un élément de filtrage FLT des réflexions parasites dont le centre définit avec le centre optique des moyens d'imagerie L1 un axe de mesure, noté z, et représenté en pointillé long. L'axe de mesure est sensiblement centré sur la pupille de l'oeil. Il comprend également des moyens de séparation optique positionnés sur l'axe de mesure et définissant la voie d'éclairage VE et la voie d'analyse VA. Dans les exemples des figures 1A, 1B et 2, les moyens de séparation sont placés en amont de l'élément de filtrage FLT et sont formés d'une première lame LM1 qui réfléchit le faisceau d'analyse FA provenant de l'oeil vers les moyens d'analyse et d'une deuxième lame LM2 qui définit la voie d'éclairage VE. Il comprend aussi, sur la voie d'éclairage, un diaphragme d'éclairage APT et des moyens de conjugaison optique, centrés sur l'axe de mesure, et permettant d'assurer la conjugaison optique entre la pupille de l'oeil PO, le plan du diaphragme d'éclairage PE et le plan d'analyse PA. Dans l'exemple simple des figures 1A et 2, ces moyens de conjugaison sont assurés par deux lentilles, L2 et L3, respectivement placées sur les voies d'analyse VA et d'éclairage VE. Le plan du diaphragme d'éclairage PE est confondu avec un plan focal de L3 et le plan d'analyse est confondu avec un plan focal de L2, permettant ainsi d'assurer la conjugaison de la pupille de l'oeil PO avec les plans PE et PA. Dans l'exemple de la figure 1B, la conjugaison du diaphragme d'éclairage PE avec la pupille de l'oeil est identique aux cas des figures 1A et 2. En revanche la conjugaison entre le plan d'analyse PA et la pupille de l'oeil est assurée par l'ensemble des deux lentilles LIF et L2, le plan d'analyse PA étant placé au foyer du système optique formé par les deux lentilles LIF et L2.

Selon l'invention, le faisceau d'éclairage FE est convergent au centre de l'élément de filtrage FLT. Dans l'exemple des figures 1A, 1B et 2, la lentille L3 assure la focalisation du faisceau d'éclairage au centre de l'élément de filtrage FLT. Par exemple, les moyens d'émission SRC sont formés d'une diode laser collimatée et l'élément de filtrage FLT se situe au foyer de la lentille L3. D'autre part, l'élément de filtrage FLT, la voie d'éclairage VE, la voie d'analyse VA, les moyens de séparation LM1, LM2, et les moyens de conjugaison L2, L3 sont solidaires, positionnés sur une plate-forme PTF1 mobile le long de l'axe de mesure (z) par rapport aux moyens d'imagerie L1. En outre, selon l'invention, le diaphragme d'éclairage APT, par exemple un diaphragme d'ouverture circulaire de diamètre prédéterminé, est décentré par rapport à l'axe de mesure z de telle sorte que le flux lumineux parasite réfléchi par la cornée de l'oeil, les moyens d'imagerie (L1), et tout dioptre situé entre l'élément de filtrage et la cornée, soit détourné de la voie d'analyse par l'élément de filtrage (FLT).

Selon la variante décrite illustrée sur la figure 1A, l'élément de filtrage FLT est formé d'une lame opaque trouée, dans cet exemple un miroir, le trou étant centré sur l'axe de mesure z, de dimensions prédéterminées de telle sorte que le faisceau d'éclairage FE et le faisceau d'analyse FA focalisés au centre dudit trou sont transmis par l'élément de filtrage tandis que les réflexions parasites sont stoppées par la partie opaque de l'élément de filtrage. Selon une variante, la taille du trou de la lame opaque est contrôlable. Une petite taille du trou permet une très bonne isolation contre les réflexions parasites formées par tout dioptre situé entre l'élément de filtrage et la cornée, en particulier lorsque le patient est équipé de verres correcteurs dont les rayons de courbure peuvent être faibles. En revanche si l'oeil mesuré n'est pas équipé de verres correcteurs et présente un fort défaut d'astigmatisme ou une pathologie de type kératocône par exemple, il est préférable que le système de mesure des aberrations n'ait pas sa dynamique de mesure limité par l'élément de filtrage de taille trop petite. Dans ce cas, la taille du trou de l'élément de filtrage peut être agrandie de façon à augmenter la dynamique de la mesure. Par ailleurs, selon l'invention et comme représenté figure 1B, l'élément de filtrage FLT peut être un élément virtuel (représenté en pointillés), image de l'élément de filtrage réel FLTO par l'élément optique LIF. Les fonctionnalité de cette variante sont identiques à celles exposées figures 1A et 2. La fonction remplie par l'objectif L2 dans le cas des figures 1A et 2, est réalisée par le système optique formé par le couple des objectifs LIF et L2 dans le cas de la figure 1B. La seule différence est que l'élément de filtrage réel FLTO se situe sur la voie d'analyse VA et que l'élément de filtrage FLT est l'image de l'élément de filtrage réel FLTO formée par la lentille LIF. L'avantage de cette variante est qu'il est plus aisé d'introduire un élément de filtrage de taille contrôlable dans le plan de FLTO que dans le plan de FLT. Dans l'exemple de la figure 2, l'élément de filtrage FLT comprend un miroir centré sur l'axe de mesure, de dimensions prédéterminées de telle sorte que le faisceau d'éclairage FE et le faisceau d'analyse FA focalisés au centre dudit miroir soient réfléchis par l'élément de filtrage tandis que les réflexions parasites ne sont pas déviées par ledit élément.

Les caractéristiques décrites ci-dessus permettent d'optimiser, en un montage compact et présentant peu d'éléments optiques, les performances du dispositif de mesure des aberrations de l'oeil. La conjugaison de la pupille de l'oeil PO avec le plan du diaphragme d'éclairage PE et le plan d'analyse PA, est indiquée sur les figures 1A, 1B et 2 en trait plein. Cette conjugaison permet d'une part, quelle que soit l'amétropie de l'oeil, que le diamètre de la zone d'éclairage sur l'oeil soit constant, permettant une maîtrise parfaite du flux incident dans l'oeil, et permet d'autre part que la taille de la zone éclairée sur le plan d'analyse PA soit directement représentative de la position et de la forme de la pupille de l'oeil, devant L1, là encore quelle que soit l'amétropie de l'oeil. Des moyens de positionnement de la pupille de l'oeil PO dans un plan focal de la lentille L1 sont pour cela nécessaires ; ils seront décrits dans la suite.

Le réglage en translation de la plate-forme PTF1 suivant l'axe de mesure z permet d'assurer, en fonction de l'amétropie de l'oeil, la conjugaison optique par la lentille L1 de la rétine de l'oeil RET avec le centre de l'élément de filtrage FLT. Il est fondamental dans le dispositif selon l'invention que l'élément de filtrage FLT, la voie d'éclairage VE, la voie d'analyse VA, les moyens de séparation (LM1, LM2), et les moyens de conjugaison (L2, L3) soient solidaires lors du déplacement de la plate-forme PTF1. En effet, cela permet de maintenir constantes les positions relatives du diaphragme d'éclairage et du plan d'analyse avec l'élément de filtrage quelle que soit la position de celui-ci, d'assurer ainsi des conditions de mesure fixes, ce qui garantit la fiabilité de la mesure. Dans l'exemple des figures 1A et 2, l'élément de filtrage FLT est sensiblement au foyer de L2 de telle sorte que le faisceau d'analyse FA arrive sensiblement collimaté sur le plan d'analyse PA. Dans le cas de la figure 1B, l'élément de filtrage FLT, image de l'élément de filtrage réel FLTO par la lentille LIF, est sensiblement au foyer du système optique de la voie d'analyse VA (formé par le couple des objectifs LIF et L2) de telle sorte que le faisceau d'analyse FA arrive sensiblement collimaté sur le plan d'analyse PA.

Sur les figures 1A, 1B et 2 est représenté, selon un exemple, le tracé du faisceau d'éclairage FE et du faisceau d'analyse FA dans le cas d'un oeil émetrope. Le faisceau sort collimaté des moyens d'émission, formés par exemple d'une diode laser émettant dans le visible ou proche infrarouge. Le diaphragme d'éclairage limite les rayons lumineux à un faisceau d'éclairage FE de diamètre prédeterminé, décentré par rapport à l'axe de mesure. L'objectif L3 focalise le faisceau FE au centre de l'élément de filtrage FLT qui, dans ce sens n'a ainsi aucune incidence sur les rayons lumineux. Après le point de focalisation le faisceau diverge et arrive sur l'objectif L1. Dans le cas d'un oeil émetrope (position "0 dioptrie" de la plate-forme PTF1), le point de focalisation au foyer de L3 (centre de l'élément de filtrage) est au foyer de L1. Le faisceau incident sur L1 est en dehors de l'axe optique compte tenu de la position excentrée du diaphragme d'éclairage, ainsi, la réflexion sur L1 (non représentée) ne revient pas avec la même direction; elle est réfléchie par le miroir troué dans la variante de la figure 1A, elle est stoppée par l'élément de filtrage FLT dans le cas de la figure 1B et elle n'est au contraire pas déviée dans l'exemple de la figure 2 où l'élément de filtrage est un petit miroir centré. A la sortie de L1, le faisceau est collimaté et arrive sur la pupille de l'oeil dans un plan image du diaphragme d'éclairage. Comme ce dernier est excentré, son image sur la pupille est aussi excentrée et la réflexion (non représentée) sur la cornée COR de l'oeil, fortement bombée, est bloquée par l'élément de filtrage. Le faisceau pénètre dans l'oeil et il est focalisé sur la rétine RET. Le choix du diamètre du diaphragme d'éclairage permet d'optimiser, compte tenu des aberrations de l'oeil, la taille du point de focalisation sur la rétine.

La rétine est un objet diffusant. Du point d'impact des rayons lumineux incidents sur la rétine sont réémis des rayons lumineux. La tache lumineuse sur la rétine sert de source d'éclairage secondaire pour les moyens d'analyse du front d'onde. La phase de l'onde diffusée par la rétine est sphérique. Issus de cette source d'éclairage secondaire, le faisceau traverse l'oeil, "enregistre" les aberrations et une partie de ce faisceau ressort de l'oeil par la pupille PO formant le faisceau d'analyse FA. Dans le cas d'un oeil emmétrope, le faisceau est globalement collimaté à la sortie de l'oeil (même vergence que le faisceau incident FE). Ce faisceau "retour" vient se focaliser au foyer de L1, au centre de l'élément de filtrage FLT, où il est confondu avec le point de focalisation du faisceau d'éclairage FE. Dans les exemples des figures 1A et 2, le centre de l'élément de filtrage est au foyer de L2 (via la lame LM1). Dans l'exemple de la figure 1B, le centre de l'élément de filtrage est sensiblement au foyer du système optique formé par les objectifs LIF et L2 (via la lame LM1). Ainsi, le faisceau analysé par l'analyseur est globalement collimaté. L'analyseur mesure les défauts de la surface d'onde générés par le système "oeil". La taille de la zone éclairée sur l'analyseur est proportionnelle à la taille de la pupille de l'oeil (au facteur de grandissement près, donné par le rapport des focales des objectifs L2 et L1 dans le cas des figures 1A et 2).

Avantageusement, la lame LM1, qui transmet le faisceau d'éclairage "aller" FE et réfléchit le faisceau d'analyse "retour" FA est une lame à faces planes et parallèles, inclinée à environ 45° sur l'axe de mesure, et dont le coefficient de réflexion est choisi pour optimiser le rendement lumineux. Par exemple, le coefficient de réflexion est supérieur à environ 70% de telle sorte à rendre maximal le flux du faisceau d'analyse (il n'est pas gênant d'avoir un coefficient de transmision faible, par exemple de l'ordre de 10%, la puissance lumineuse des moyens d'émission pouvant être ajustée en conséquence). Cette configuration en réflexion présente en outre l'avantage de ne pas introduire d'astigmatisme sur le faisceau d'analyse FA (sur un faisceau non collimaté, en transmission, une lame à faces planes et parallèles introduit de l'astigmatisme). Il est également intéressant de choisir l'épaisseur de la lame LM1 pour que le décalage introduit sur le faisceau d'éclairage par rapport à l'axe de mesure z soit tel que si la lame LM1 n'est plus en place, pour une raison accidentelle, entraînant une augmentation brusque du flux incident dans l'oeil, le faisceau d'éclairage se trouve stoppé (cas de la figure 1A), ou non réfléchi vers l'oeil (cas de la figure 2) par l'élément de filtrage.

Dans les exemples décrits ci-dessus, les moyens de positionnement de l'oeil comprennent une voie d'imagerie VI de la pupille, avec un système d'imagerie IMA solidaire de la plate-forme PTF1, et comprenant un détecteur DET situé dans un plan focal d'un objectif L4. Un dispositif d'éclairage ECL, par exemple formé de diodes électroluminescentes ECL, peut être prévu pour illuminer la pupille de l'oeil. Le positionnement de la pupillle est ajusté par exemple en contrôlant la mise au point de l'image de la pupille sur le détecteur DET. Avantageusement, le dispositif d'éclairage de l'oeil étant formé d'un ensemble de sources lumineuses, le positionnement peut être ajusté en contrôlant la mise au point de l'image desdites sources réfléchies par la cornée sur le détecteur, permettant ainsi un contrôle du positionnement du plan tangent au sommet de la cornée de l'oeil. Il est préférable d'avoir un contrôle sur la position de ce plan plutôt que sur la position du plan de la pupille car l'amétropie de l'oeil est définie par rapport au sommet de la cornée.

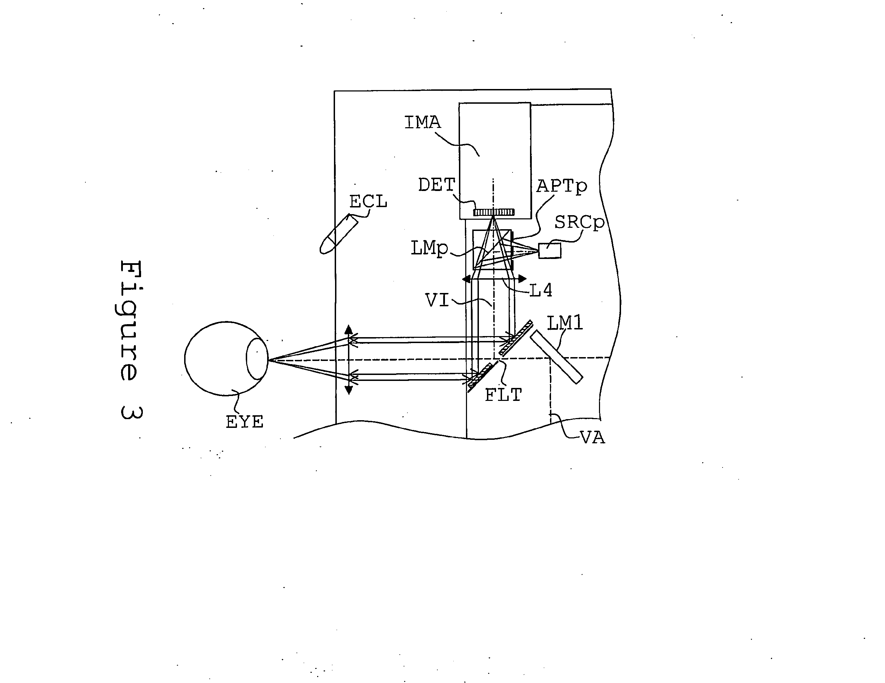

Pour affiner encore le réglage de la position du plan tangent au sommet de la cornée par rapport aux moyens d'imagerie L1, on peut prévoir un système d'aide au positionnement tel qu'il est décrit sur la figure 3. La figure 3 reprend l'exemple de montage décrit sur la figure 1A mais par souci de simplicité, seuls certains éléments sont représentés. Le système d'aide au positionnement comprend une source lumineuse quasi-ponctuelle SRCp, centrée sur l'axe de mesure z, et positionnée sensiblement au foyer de l'objectif L4 de la voie d'imagerie, un masque formé d'au moins deux ouvertures dont au moins une est décentrée par rapport à l'axe de mesure, et positioné à proximité de ladite source, pour former au moins deux faisceaux lumineux, et une lame séparatrice $LM_P$ recevant lesdits faisceaux. Le positionnement est ajusté en contrôlant sur le détecteur DET de la voie d'imagerie, la superposition des taches formées par les faisceaux après réflexion sur la cornée de l'oeil, permettant ainsi un contrôle du positionnement du plan tangent au sommet de la cornée de l'oeil. Dans cet exemple, deux faisceaux sont formés grâce à à un double diaphragme $APT_P$ placé à l'entrée d'un cube séparateur (côté source) dont la face sépratrice forme la lame séparatrice. La source peut être formée d'une diode laser ou d'une diode électroluminescente.

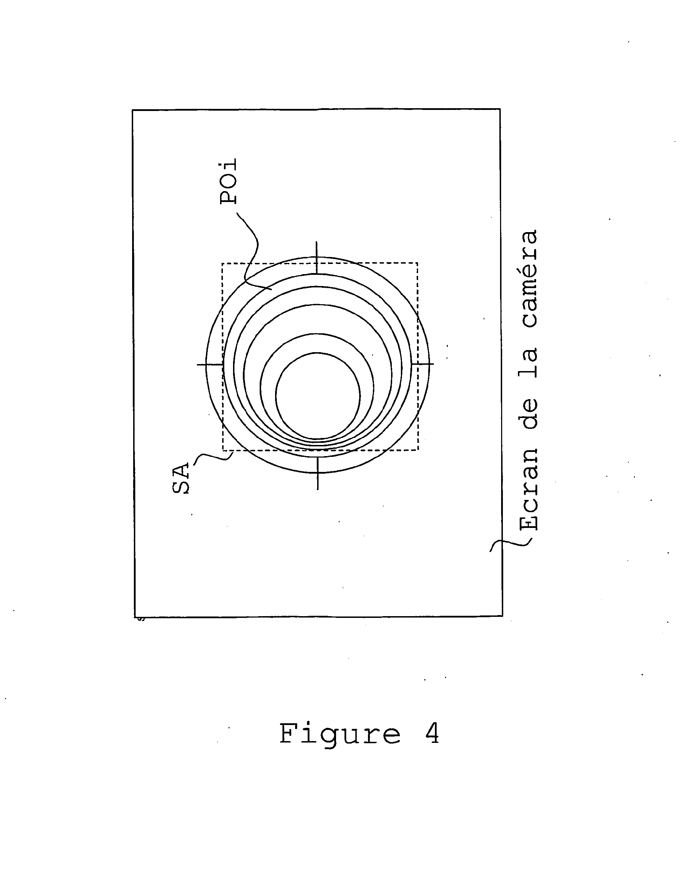

Selon une variante, les moyens de positionnement de l'oeil comprennent en outre sur l'affichage de la caméra de la voie d'imagerie, des moyens de repérage de la position latérale de la pupille de l'oeil, permettant d'ajuster cette position latérale par rapport à la position du faisceau d'éclairage en fonction de la taille de la pupille. Ces moyens de repérage, qui apparaissent par exemple sur l'affichage de la caméra de la voie d'imagerie, sont représentés, selon un exemple, sur la figure 4. Le carré en pointillé SA représente la surface utile de l'analyseur rapportée dans le plan de la caméra d'imagerie IMA de la voie d'imagerie VI (figure 1A ou 2). Les

cercles PO$_i$ représentent les repères de positionnement latéral pour des pupilles de taille différente. Lorsque l'oeil présente une taille de pupille importante (sujet jeune ou oeil sous produit mydriatique), il faut centrer au mieux la pupille de façon à ce que la surface couverte sur l'analyseur soit optimale. Compte tenu de la grande taille de la pupille, il n'y a pas de problème pour que le faisceau incident FE (sur la figure 1A ou 2 par exemple), décalé de l'axe optique, pénètre dans la pupille de l'oeil étudié. En revanche, lorsque la pupille de l'oeil est plus petite (oeil âgé typiquement), il faut l'excentrer pour que le flux incident rentre dans l'oeil.

Avantageusement, les moyens de positionnement comprennent en outre des moyens de déplacement des moyens d'imagerie L1 et de la plate-forme PTF1, solidairement, pour procéder à l'ajustement du positionnement de la pupille. Dans les exemples des figures 1A, 1B et 2, l'objectif L1 et la plate-forme PTF1 sont montés sur une seconde plate-forme PTF2, qui peut se déplacer dans toutes les directions.

Dans l'exemple de la figure 1A, le trou de filtrage FLT des réflexions parasites est formé dans un miroir réfléchissant MIR, incliné par rapport à l'axe de mesure z d'environ 45°, envoyant vers la voie d'imagerie VI le flux lumineux issu de la pupille PO. L'utilisation du miroir MIR ainsi disposé permet notamment de ne pas ajouter de lame supplémentaire qui serait traversée par le faisceau d'analyse. Dans l'exemple de la figure 2, le miroir centré laisse passer le flux lumineux issu de la pupille PO vers la voie d'imagerie VI.

Comme cela apparaît sur la figure 1A par exemple, le dispositif selon l'invention peut également comprendre une voie de fixation VF, permettant de fixer l'attention du patient dont l'oeil est analysé, et comprenant une image éclairée FIX formant une cible de fixation et un objectif L5, l'objectif assurant sensiblement la conjugaison optique de l'image avec le trou de filtrage FLT (le tracé des rayons n'est pas représenté sur la figure 1A par souci de clarté). Idéalement, la cible de fixation n'est pas parfaitement conjuguée avec le trou de filtrage et la rétine ; on règle le système de manière à ce que cette cible soit vue avec une légère défocalisation myopique qui stimule la désaccomodation.

De manière pratique, le protocole de mesure des aberrations avec le dispositif selon l'invention peut être le suivant. Si le patient connaît approximativement son amétropie (il est soit myope, soit hypermétrope, soit emmetrope), on règle la position de la plate-forme PTF1 à environ 2 dioptries au-dessus de l'amétropie annoncée pour s'assurer que le patient ne puisse pas accomoder (pour un oeil émetrope, on règle par exemple la plate-forme PTF1 à "+ 2 dioptries", c'est-à-dire que le trou de filtrage FLT est éloigné par rapport à l'objectif L1 d'une distance permettant de conjuguer la rétine et le trou de filtrage par L1 dans le cas où l'oeil aurait une hypermétropie de 2 dioptries). Grâce à la caméra d'imagerie IMA de la voie d'imagerie VI, on ajuste la position de la plate-forme PTF2 par rapport à l'oeil pour assurer la conjugaison par exemple du plan tangent au sommet de la cornée de l'oeil PO avec le plan du diaphragme d'éclairage PE et le plan d'analyse PA. Une première mesure avec les moyens d'analyse du front d'onde permet de déterminer sur le front d'onde la défocalisation globale du faisceau d'analyse. Avantageusement, le dispositif selon l'invention comprend en outre des moyens de commande COM de la plate-forme PTF1, reliés aux moyens d'analyse MA, et permettant de commander le déplacement selon l'axe de mesure z de la plate-forme PTF1. Les moyens de commande reçoivent des moyens de traitement TRT la valeur de la défocalisation globale sur le faisceau d'analyse et peuvent commander le déplacement de la plate-forme PTF1 en conséquence, de telle sorte à assurer la conjugaison de la rétine de l'oeil avec l'élément de filtrage FLT par l'objectif L1. La valeur de ce déplacement par rapport à une position d'origine permet de déterminer avec précision l'amétropie réelle de l'oeil. On procède alors à la mesure des aberrations. Il est souhaitable de régler la plate-forme PTF1 lors de cette mesure à une position correspondant à quelques fractions de dioptrie au-dessus de l'amétropie réelle de l'oeil afin de s'assurer que le patient ne vas pas accommoder pendant la mesure.

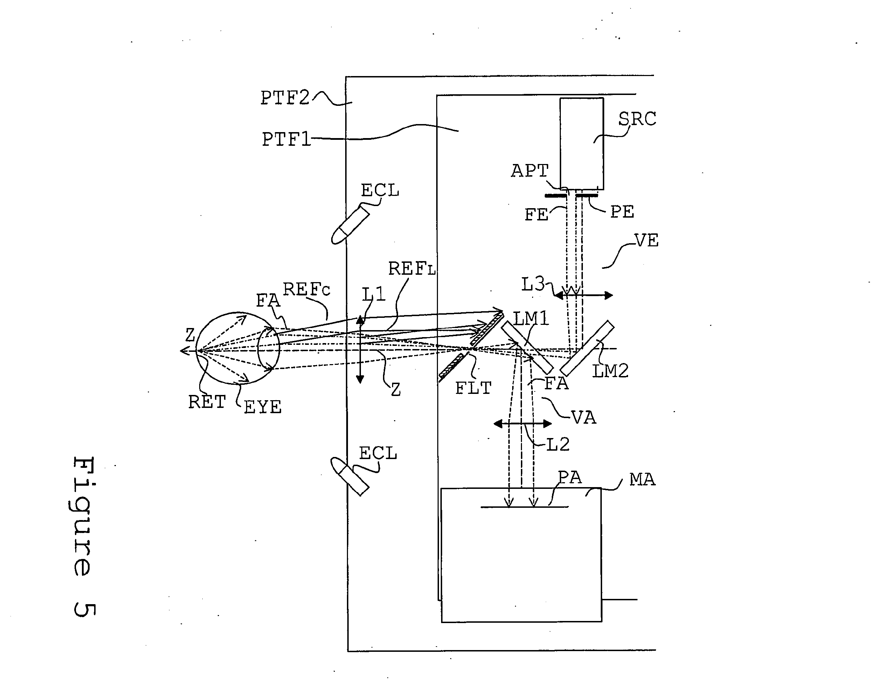

La figure 5 représente ainsi le tracé des rayons dans le cas de l'analyse des aberrations d'un oeil myope. Sur ce schéma, par souci de clarté, seules sont représentées les voies d'analyse et d'éclairage, mais le montage est le même que dans l'exemple de la figure 1A. Dans cet exemple, compte tenu du fait que l'oeil est myope, le faisceau d'analyse "retour" FA est globalement convergent à la sortie de l'oeil. Comme cela a été décrit précédemment, les moyens d'analyse du front d'onde permettent de déterminer la valeur du déplacement de la plate-forme PTF1 pour assurer. la conjugaison de la rétine de l'oeil RET avec le trou de filtrage FLT par l'objectif L1. En pratique, la valeur de déplacement Dep (le sens positif est indiqué par une flèche sur l'axe de mesure z) de la plate-forme PTF1 en fonction de l'amétropie de l'oeil (en dioptrie) est:

$$Dep = - f_1{}^2 \times D$$

Où $f_1$ est la distance focale de l'objectif L1. Dans l'exemple d'un oeil myope, la plate-forme PTF1 est rapprochée de l'objectif L1.

La figure 5 permet de mettre en évidence l'élimination des réflexions parasite grâce au dispositif selon l'invention. Le montage représenté est identique à celui de la figure 1A. En effet, la mise en oeuvre d'un faisceau d'éclairage FE décentré par rapport à l'axe de mesure z en association avec le trou de filtrage tel qu'il a été décrit permet une élimination très efficace non seulement des réflexions parasites sur la cornée (réflexions notées REF$_C$ sur la figure 5) mais également des réflexions parasites sur les moyens d'imagerie L1 (réflexions notées REF$_L$ sur la figure 5), et cela sans perte du flux utile pour l'analyse.

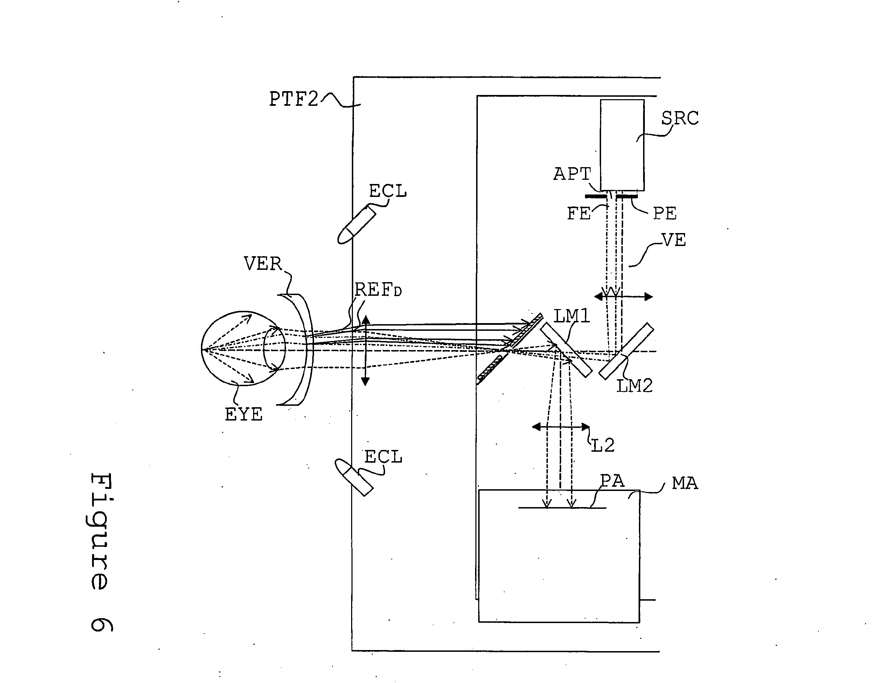

Il est remarquable de noter que le dispositif selon l'invention permet également de s'affranchir des réflexions parasites de tout dioptre placé entre l'oeil et l'élément de filtrage FLT, et notamment de verres de lunettes, comme cela est illustré sur la figure 6. En effet, les verres de lunettes (VER) se présentent comme deux dioptres bombés, les réflexions parasites (notées REF$_u$ sur la figure 6) sont donc stoppées par le trou de filtrage FLT de la même façon que les autres réflexions parasites. Cela permet de pouvoir procéder à l'analyse de l'oeil d'un patient équipé de ses verres correcteurs et de vérifier ainsi l'ensemble des aberrations du système oeil et verres correcteurs, ce qui constitue un avantage important du dispositif selon l'invention. Sur le montage de la figure 6, on peut noter que la plate-forme PTF1 est de nouveau en position "0 dioptrie" puisque les verres correcteurs permettent de corriger de la myopie.

Grâce à ses performances en matière de suppression des réflexions parasites, le dispositif selon l'invention permet en outre l'analyse d'un système de type oeil artificiel, dans lequel on voudrait par exemple tester la qualité d'un implant. En effet, dans un tel système, les réflexions parasites sont accrues du fait d'un plus grand nombre d'interfaces air/matière (dans un oeil vivant, la réflexion parasite se produit principalement sur la cornée).

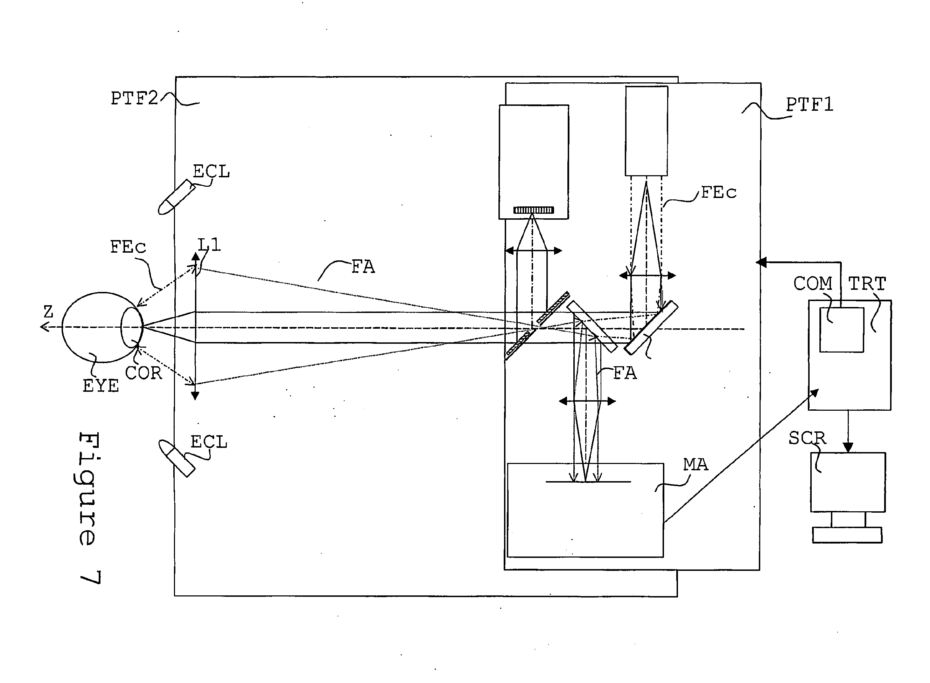

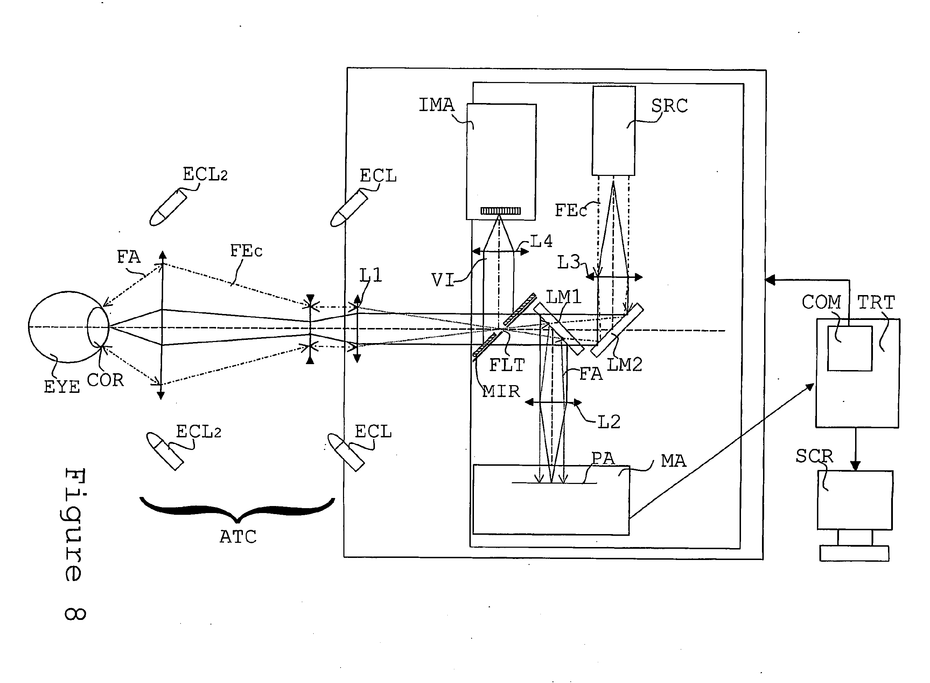

Le dispositif selon l'invention permet également de procéder à la mesure de la topographie de la face antérieure de la cornée COR de l'oeil, avec tous les avantages précédemment décrits. Cette mesure permet notamment de connaître avec précision quelle est la part de la forme de la surface antérieure de la cornée dans les aberrations de l'oeil. Elle permet également un contrôle de cette surface, par exemple en chirurgie oculaire pour la correction de la vision. Deux exemples de montage permettant cette mesure sont illustrés sur les figures 7 et 8.

La figure 7 présente un montage sensiblement similaire à celui de la figure 1A. Cependant, la voie d'éclairage comprend en outre des moyens d'émission d'un faisceau d'éclairage FE$_C$ centré sur l'axe de mesure z, de diamètre prédéterminé, convergent au centre de l'élément de filtrage FLT. En pratique, il peut s'agir d'un faisceau émis par la même source SRC que dans l'exemple de la figure 1A mais le diaphragme APT a été retiré de manière à centrer le faisceau d'éclairage et à agrandir son diamètre. Pour procéder à la mesure de la topographie de la cornée, la position de la plate-forme PTF1 est ajustée le long de l'axe de mesure z (en pratique, la plate-forme est éloignée) de manière à assurer sensiblement la conjugaison optique par les moyens d'imagerie L1 du centre de courbure de la cornée avec le centre de l'élément de filtrage FLT, permettant ainsi l'autocollimation du faisceau d'éclairage sur la face antérieure de la cornée. Le flux réfléchi FA sur ladite face est alors envoyé vers les moyens d'analyse MA afin de procéder à la mesure. Cette configuration nécessite de travailler avec un objectif L1 de courte focale et de grande ouverture et nécessite par ailleurs de prévoir une course de déplacement importante pour la plate-forme PTF1. L'augmentation du chemin optique entre l'élément de filtrage et l'objectif L1 peut aussi être réalisée par l'intermédiaire d'un jeu de 3 miroirs ou plus.

Dans la variante illustrée sur la figure 8, ces contraintes sont évitées en ajoutant un système d'autocollimation additionnel ATC entre l'objectif L1 et l'oeil EYE, ce système étant solidaire des moyens d'imagerie L1. Le système d'autocollimation permet de focaliser le faisceau d'éclairage FE$_c$ au centre de courbure de la cornée, le flux réfléchi sur la face antérieure de la cornée étant envoyé vers les moyens d'analyse MA afin de procéder à la mesure de la topographie de la cornée.

Les autres caractéristiques des montages des figures 7 ou 8 sont identiques à celles du montage de la figure 1A. Notamment, le faisceau d'éclairage FE$_c$ est focalisé grâce à l'objectif L3 au centre de l'élément de filtrage FLT, puis repris par l'objectif L1 dont un foyer est sensiblement confondu avec le centre de l'élément de filtrage. Le faisceau d'éclairage FE$_c$ arrive sensiblement collimaté sur le système d'autocollimation ATC. Celui-ci est calculé pour une cornée "standard", de rayon de courbure donné de telle sorte que le faisceau d'éclairage incident sur ladite cornée standard soit convergent au centre de courbure de la cornée, le faisceau réfléchi FA sur la cornée (correspondant aux 4% environ de réflexion) étant alors confondu avec le faisceau incident FE. Le faisceau FA est alors analysé comme précédemment par les moyens d'analyse MA afin de déterminer la phase du front de l'onde réfléchie par la cornée, ce qui permet de calculer ses paramètres de forme et d'en établir une cartographie. Il est nécessaire comme dans le cas de mesure de l'esemble des aberrations de l'oeil, d'assurer la conjugaison entre un plan prédéterminé de l'oeil (plan de la pupille ou avantageusement, plan tangent au sommet de la cornée) et le plan d'analyse PA. Pour cela, on peut utiliser des moyens de positionnement de l'oeil tels qu'ils ont été décrits précédemment, en utilisant la voie d'imagerie VI. La plate-forme 1, l'objectif L1 et le système d'autocollimation ATC sont déplacés solidairement le long de l'axe de mesure z jusqu'à ce que par exemple, l'image de la pupille soit nette sur la caméra d'imagerie IMA. Si la cornée est "standard", le faisceau d'analyse FA resort en faisceaux parallèles du système d'autocollimation, est focalisé au centre du trou de filtrage par L1 puis est envoyé vers les moyens d'analyse MA par la lame LM1 où il est incident en ondes planes. Si la cornée présente un écart de rayon de courbure par rapport à une cornée standard, celui-ci est mesuré par les moyens d'analyse. Une mesure très précise de ce rayon de courbure est possible en procédant à des déplacements de la plate-forme PTF1 de part et d'autre de la position pour laquelle le faisceau d'analyse est incident sur les moyens d'analyse en ondes planes. Pour cette mesure, les réflexions parasites sur les moyens d'imagerie sont beaucoup moins critiques. En effet, la réflexion sur la cornée génère un flux lumineux beaucoup plus important que la diffusion sur la rétine de l'oeil et un traitement antireflet sur les moyens d'imagerie suffit à limiter l'effet des réflexions parasites, malgré que le faisceau d'éclairage ne soit pas décentré.

## Revendications

1. Dispositif de mesure des aberrations d'un système de type oeil, comprenant une voie d'éclairage avec notamment des moyens d'émission d'un faisceau d'éclairage (FE) pour former par rétrodiffusion sur la rétine de l'oeil une source lumineuse secondaire et une voie d'analyse avec notamment des moyens d'analyse dans un plan d'analyse (PA) donné de la phase du front de l'onde émise par ladite source secondaire et émergente de l'oeil, comprenant en outre:

   - des moyens d'imagerie (L1) et des moyens de positionnement de l'oeil permettant notamment le positionnement d'un plan prédéterminé de l'oeil dans un plan focal desdits moyens d'imagerie,
   - un élément de filtrage (FLT) des réflexions parasites dont le centre définit avec le centre optique des moyens d'imagerie (L1) un axe de mesure (z), sensiblement centré sur la pupille de l'oeil,
   - des moyens de séparation optique (LM1) positionnés sur l'axe de mesure et définissant la voie d'éclairage et la voie d'analyse,
   - sur la voie d'éclairage, un diaphragme d'éclairage (APT) d'ouverture prédéterminée,
   - des moyens de conjugaison optique (L2, L3) centrés sur l'axe de mesure et permettant d'assurer la conjugaison optique entre ledit plan prédéterminé de l'oeil, le plan du diaphragme d'éclairage et le plan d'analyse,
   et **caractérisé en ce que** le faisceau d'éclairage est convergent au centre de l'élément de filtrage (FLT), **en ce que** l'élément de filtrage, la voie d'éclairage, la voie d'analyse, les moyens de séparation et les moyens de conjugaison sont solidaires, positionnés sur une plate-forme (PTF1) mobile par rapport aux moyens d'imagerie (L1) le long de l'axe optique desdits moyens (z), permettant le réglage, en fonction de l'amétropie de l'oeil, de la conjugaison optique par lesdits moyens d'imagerie (L1) de la rétine de l'oeil avec le centre de l'élément de filtrage, et **en ce que** le diaphragme d'éclairage est décentré par rapport à l'axe de mesure cm à tout axe conjugué de l'axe de mesure de telle sorte que le flux lumineux parasite réfléchi par la cornée de l'oeil, les moyens d'imagerie (L1), et tout dioptre situé entre l'élément de filtrage et la cornée, soit détourné de la voie d'analyse par l'élément de filtrage (FLT).

2. Dispositif selon la revendication 1, **caractérisé en ce que** l'élément de filtrage (FLT) est formé d'une lame opaque trouée, le trou étant centré sur l'axe de mesure (z), de dimensions prédéterminées de telle sorte que le faisceau d'éclairage (FE) et le faisceau d'analyse (FA) focalisés sensiblement au centre dudit trou sont transmis par l'élément de filtrage tandis que les réflexions parasites sont stoppées par la partie opaque de l'élément de filtrage.

3. Dispositif selon la revendication 1, **caractérisé en ce que** l'élément de filtrage (FLT) est virtuel, formé par l'image d'un élément de filtrage réel (FLTO), ledit élément de filtrage réel (FLTO) étant placé sur la voie d'analyse VA.

4. Dispositif selon la revendication 3, **caractérisé en ce que** l'élément de filtrage réel (FLTO) est formée d'une lame opaque trouée.

5. Dispositif selon les revendications 2 ou 4, **caractérisé en ce que** la taille du trou de la lame opaque soit contrôlable.

6. Dispositif selon la revendication 1, **caractérisé en ce que** l'élément de filtrage (FLT) est formé d'un miroir troué, le trou étant centré sur l'axe de mesure (z), de dimensions prédéterminées de telle sorte que le faisceau d'éclairage (FE) et le faisceau d'analyse (FA) focalisés sensiblement au centre dudit trou sont transmis par l'élément de filtrage tandis que les réflexions parasites sont déviées par la partie réfléchissante de l'élément de filtrage.

7. Dispositif selon la revendication 1, **caractérisé en ce que** l'élément de filtrage (FLT) comprend un miroir centré sur l'axe de mesure, de dimensions prédéterminées de telle sorte que le faisceau d'éclairage (FE) et le faisceau d'analyse (FA) focalisés sensiblement au centre dudit miroir sont réfléchis par l'élément de filtrage tandis que les réflexions parasites ne sont pas déviées par ledit élément.

8. Dispositif selon l'une des revendications précédentes, **caractérisé en ce qu'**il comprend en outre des moyens de commande (COM) de ladite plate-forme (PTF1), reliés aux moyens d'analyse, entraînant un déplacement selon l'axe de mesure (z) de la plate-forme suivant une fonction prédéterminée de l'amétropie de l'oeil mesurée par lesdits moyens d'analyse.

9. Dispositif selon l'une des revendications précédentes, **caractérisé en ce que** lesdits moyens de positionnement de l'oeil comprennent notamment une voie d'imagerie (VI) de la pupille de l'oeil (P0), avec un objectif (L4) sensiblement centré sur l'axe de mesure (z) et une caméra d'imagerie solidaire de ladite plate-forme (PTF1) comprenant un détecteur situé dans un plan focal dudit objectif (L4) et un système d'affichage.

10. Dispositif selon la revendication 9, **caractérisé en ce que** les moyens de positionnement comprennent en outre un dispositif d'éclairage de l'oeil formé d'un ensemble de sources lumineuses, le positionnement étant ajusté en contrôlant la mise au point de l'image desdites sources réfléchies par la cornée sur le détecteur, permettant ainsi un contrôle du positionnement du plan tangent au sommet de la cornée de l'oeil.

11. Dispositif selon l'une des revendications 9 ou 10, **caractérisé en ce que** les moyens de positionnement comprennent en outre une source lumineuse quasi-ponctuelle, centrée sur l'axe de mesure (z), et positionnée sensiblement au foyer dudit objectif (L4), un masque formé d'au moins deux ouvertures dont au moins une est décentrée par rapport à l'axe de mesure, et positionné à proximité de ladite source, pour former au moins deux faisceaux lumineux, une lame séparatrice recevant lesdits faisceaux, le positionnement étant ajusté en contrôlant sur le détecteur de la voie d'imagerie, la superposition des taches formées par lesdits faisceaux après réflexion sur la cornée de l'oeil, permettant ainsi un contrôle du positionnement du plan tangent au sommet de la cornée de l'oeil.

12. Dispositif selon l'une des revendications 9 à 11, **caractérisé en ce que** les moyens de positionnement de l'oeil comprennent en outre sur l'affichage du détecteur de la voie d'imagerie, des moyens de repérage de la position latérale de la pupille de l'oeil, permettant d'ajuster ladite position par rapport à la position du faisceau d'éclairage en fonction de la taille de la pupille.

13. Dispositif selon l'une des revendications 9 à 12, **caractérisé en ce que** lesdits moyens de positionnement comprennent en outre des moyens de déplacement des moyens d'imagerie (L1) et de la plate-forme (PTF1), solidairement, pour procéder à l'ajustement du positionnement de l'oeil.

14. Dispositif selon l'une des revendications précédentes, **caractérisé en ce que** les moyens de conjugaison optique comprennent des moyens d'imagerie (L2) sur la voie d'analyse, le plan d'analyse étant confondu avec un plan focal desdits moyens d'imagerie, et des moyens d'imagerie (L3) sur la voie d'éclairage, le plan du diaphragme d'éclairage étant confondu avec un plan focal desdits moyens d'imagerie, lesdits moyens (L3) assurant également la focalisation du faisceau d'éclairage au centre du l'élément de filtrage (FLT).

15. Dispositif selon l'une des revendications précédentes, **caractérisé en ce que** les moyens de séparation comprennent au moins une première lame séparatrice (LM1), sensiblement à faces planes et parallèles, inclinée sur l'axe de mesure (z), traversée par une partie du faisceau d'éclairage et réfléchissant une partie de l'onde émergente de l'oeil vers la voie d'analyse.

16. Dispositif selon la revendication 15, **caractérisé en ce que** le coefficient de réflexion de ladite lame (LM1) est supérieur à environ 70%.

17. Dispositif selon l'une des revendications 15 ou 16, **caractérisé en ce que** l'épaisseur de ladite lame (LM1) introduit sur le faisceau d'éclairage un décalage prédéterminé par rapport l'axe de mesure (z), de telle sorte qu'en cas d'absence accidentelle de ladite lame, le faisceau d'éclairage ne passe plus à travers le l'élément de filtrage.

18. Dispositif selon l'une des revendications précédentes, **caractérisé en ce qu'**il comprend en outre une voie de fixation, permettant de fixer la direction du regard du patient dont l'oeil est analysé, et comprenant une image éclairée et un objectif (L5), l'objectif assurant sensiblement la conjugaison optique de l'image avec l'élément de filtrage (FLT).

19. Dispositif selon l'une des revendications précédentes, **caractérisé en ce que** les moyens d'analyse comprennent un analyseur de front d'onde de type Shack-Hartmann comprenant notamment une matrice de micro-lentilles, un détecteur matriciel, des moyens de traitement, le plan d'analyse correspondant au plan de la matrice de microlentilles.

20. Dispositif selon l'une des revendications précédentes, **caractérisé en ce que** la voie d'éclairage comprend en outre des moyens d'émission d'un faisceau d'éclairage centré sur l'axe de mesure (z), de diamètre prédéterminé, convergent au centre de l'élément de filtrage (FLT), et **en ce que** la position de la plate-forme (PTF1) est ajustée le long de l'axe de mesure (z) pour assurer sensiblement la conjugaison optique par les moyens d'imagerie (L1) du centre de courbure de la cornée avec le centre de l'élément de filtrage (FLT), permettant l'autocollimation dudit faisceau d'éclairage sur la face antérieure de la cornée, le flux réfléchi sur ladite face étant envoyé vers les moyens d'analyse (MA) afin de procéder à la mesure de la topographie de la cornée.

**21.** Dispositif selon l'une des revendications 1 à 19, **caractérisé en ce que** la voie d'éclairage comprend en outre des moyens d'émission d'un faisceau d'éclairage çentré sur l'axe de mesure (z), de diamètre prédéterminé, convergent au centre de l'élément de filtrage (FLT), et **en ce que** les moyens d'imagerie comprennent un système d'autocollimation additionnel et amovible permettant de focaliser ledit faisceau d'éclairage (FE) au centre de courbure de la cornée, le flux réfléchi sur la face antérieure de la cornée étant envoyé vers les moyens d'analyse (MA) afin de procéder à la mesure de la topographie de la cornée.

**Claims**

**1.** A device for measuring the aberrations in an eye-type system, comprising an illumination path with notably means for transmitting an illumination beam (FE) to form by backscattering on the retina of the eye a secondary light source and a test path with notably test means in a given test plane (PA) of the phase of the front of the wave emitted by said secondary source and emerging from the eye, comprising moreover:

- imaging means (L1) and eye-positioning means enabling in particular to position a predetermined plane of the eye in a focal plane of said imaging means,
- a stray reflection filtering element (FLT) whereof the centre defines with the optical centre of the imaging means (L1) a measurement axis (z), substantially centred on the pupil of the eye,
- optical separation means (LM1) placed on the measurement axis and defining the illumination path and the test path,
- on the illumination path, an illumination diaphragm, of predetermined aperture,
- optical conjugation means (L2, L3) centred on the measurement axis and enabling optical conjugation between said predetermined plane of the eye, the plane of the illumination diaphragm and the test plane,

and **characterised in that** the illumination beam converges at the centre of the filtering element (FLT), **in that** the filtering element, the illumination path, the test path, the separation means and the conjugation means are interdependent, placed on a platform (PTF1) mobile relative to the imaging means (L1) along the optical axis of said means (z), enabling adjustment, with respect to the ametropia of the eye, of the optical conjugation by said means (L1) of the retina of the eye with the centre of the filtering element, and **in that** the illumination diaphragm is off-centre with respect to the measurement axis or any conjugate axis of the measurement axis, so that the stray light flux reflected by the cornea of the eye, the imaging means (L1) as well as any diopter situated between the filtering element and the cornea, is deflected from the test path by the filtering element (FLT).

**2.** A device according to claim 1, **characterised in that** the filtering element (FLT) is composed of a drilled opaque blade, the hole being centred on the measurement axis (z), of predetermined dimensions so that the illumination beam (FE) and the test beam (FA) both focussed substantially at the centre of said hole, are transmitted by the filtering element whereas the stray reflections are stopped by the opaque portion of the filtering element.

**3.** A device according to claim 1, **characterised in that** the filtering element (FLT) is virtual, formed by the image of a real filtering element (FLTO), said real filtering element (FLTO) being placed on the test path VA.

**4.** A device according to claim 3, **characterised in that** the real filtering element (FLTO) is formed of a drilled opaque blade.

**5.** A device according to claim 2 or 4, **characterised in that** the size of the hole of the opaque blade is controllable.

**6.** A device according to claim 1, **characterised in that** the filtering element (FLT) is composed of a drilled mirror, the hole being centred on the measurement axis (z), of predetermined dimensions so that the illumination beam (FE) and the test beam (FA) both focussed substantially at the centre of said hole, are transmitted by the filtering element whereas the stray reflections are deflected by the reflecting portion of the filtering element.

**7.** A device according to claim 1, **characterised in that** the filtering element (FLT) comprises a mirror centred on the measurement axis, of predetermined dimensions so that the illumination beam (FE) and the test beam (FA) both focussed substantially at the centre of said mirror, are reflected by the filtering element whereas the stray reflections are not deflected by said element.

**8.** A device according to one of the previous claims, **characterised in that** it comprises moreover means (COM) for controlling said platform (PTF1), connected to the test means, causing a displacement along the measurement axis (z) of the platform according to a predetermined function of the ametropia of the eye measured by said test means.

9. A device according to one of the previous claims, **characterised in that** said eye-positioning means comprise notably an imaging path (VI) of the pupil of the eye (PO), with a lens (L4) substantially centred on the measurement axis (z) and an imaging camera interdependent upon said platform (PTF1) comprising a detector situated on a focal plane of said lens (L4) and a display system.

10. A device according to claim 9, **characterised in that** the positioning means comprise moreover an illumination device of the eye formed of a set of light sources, whereas the position can be adjusted by tuning the focussing of the image of said sources reflected by the cornea on the detector, thereby enabling to control the position of the plane tangent to the apex of the cornea of the eye.

11. A device according to any of the claims 9 or 10, **characterised in that** the positioning means comprise moreover a quasi-punctual light source, centred on the measurement axis (z), and placed substantially at the focus of said lens (L4), a mask formed of at least two apertures whereof at least one is off-centre with respect to the measurement axis, and located near said source, to form at least two light beams, a separating blade receiving said beams, the position being adjusted by controlling on the detector of the imaging path, the superimposition of the spots formed by the beams after reflection on the cornea of the eye, thus enabling controlled positioning of the plane tangent to the apex of the cornea of the eye.

12. A device according to one of the claims 9 to 11, **characterised in that** the eye-positioning means comprise moreover on the display of the detector of the imaging path, means for tracking the lateral position of the pupil of the eye, enabling to adjust this lateral position with respect to the position of the illumination beam relative to the size of the pupil.

13. A device according to one of the claims 9 to 12, **characterised in that** said positioning means comprise moreover means for moving the imaging means (L1) and the platform (PTF1), interdependently, in order to adjust the position of the eye.

14. A device according to one of the previous claims, **characterised in that** the optical conjugation means comprise imaging means (L2) on the test path, the test plane being coincident with a focal plane of said imaging means, and imaging means (L3) on the illumination path, the plane of the illumination diaphragm being coincident with a focal plane of said imaging means, said means (L3) also focussing the illumination beam at the centre of the filtering element (FLT).

15. A device according to one of the previous claims, **characterised in that** the separation means comprise at least a first separating blade (LM1), substantially with flat and parallel faces, tilted on the measurement axis (z), crossed by a portion of the illumination beam and reflecting a portion of the wave emerging from the eye towards the test path.

16. A device according to claim 15, **characterised in that** the reflection coefficient of said blade (LM1) is greater than approximately 70 %.

17. A device according to one of the claims 15 or 16, **characterised in that** the thickness of said blade (LM1) introduces on the illumination beam, a predetermined offset with respect to the measurement axis (z), so that failing said blade accidentally, the illumination beam does not go through the filtering element any longer.

18. A device according to one of the previous claims, **characterised in that** it comprises moreover a fastening path, enabling to fix the direction of the patient's gaze whereof the eye is tested, and including an illuminated image and a lens (L5), the lens substantially optically conjugating the image with the filtering element (FLT).

19. A device according to one of the previous claims, **characterised in that** the test means comprise a Shack-Hartmann type wave-front analyser including notably a matrix of microlenses, a matrix detector, processing means, and the test plane corresponding to the plane of the microlens matrix.

20. A device according to one of the previous claims, **characterised in that** the illumination path comprises moreover means for transmitting an illumination beam centred on the measurement axis (z), of predetermined diameter, converging at the centre of the filtering element (FLT), and **in that** the position of the platform (PTF1) is adjusted along the measurement axis (z) for substantially optical conjugation by the imaging means (L1) of the curvature centre of the cornea with the centre of the filtering element (FLT), enabling self-collimation of said illumination beam on the anterior face of the cornea, the flux reflected on said face being sent towards the test means (MA) in order to measure the topography of the cornea.

21. A device according to one of the claims 1 to 19, **characterised in that** the illumination path comprises moreover means for transmitting an illumination beam centred on the measurement axis (z), of predetermined diameter, converging at the centre of the filtering element (FLT), and **in that** the imaging means comprise an additional and removable self-collimation system, enabling to focus said illumination beam (FE) at the curvature centre of the cornea, the flux reflected on the anterior face of the cornea being sent towards the test means (MA) in order to measure the topography of the cornea.

**Patentansprüche**

1. Vorrichtung zur Messung von Aberrationen in einem augenartigen System, enthaltend einen Lichtweg insbesondere mit Mitteln zum Aussenden eines Lichtstrahls (FE), um durch Rückstreuung auf der Netzhaut des Auges eine sekundäre Lichtquelle zu bilden, und einen Analyseweg insbesondere mit Mitteln zur Analyse der Wellenfrontphase der von der sekundären Quelle ausgesendeten und aus dem Auge austretenden Welle in einer gegebenen Analyseebene (PA), welche Vorrichtung ferner enthält:

   - Abbildungsmittel (L1) und Mittel zum Positionieren des Auges, mit denen insbesondere die Positionierung einer vorbestimmten Ebene des Auges in einer Fokalebene der Abbildungsmittel erfolgen kann,
   - ein Filterelement (FLT) zum Ausfiltern von Störreflexionen, dessen Mitte zusammen mit dem optischen Mittelpunkt der Abbildungsmittel (L1) eine Messachse (z) definiert, die im wesentlichen auf die Pupille des Auges zentriert ist,
   - Mittel (LM1) zur optischen Trennung, die auf der Messachse positioniert sind und den Lichtweg und den Analyseweg definieren,
   - auf dem Lichtweg eine Lichtblende (APT) mit vorbestimmter Öffnung,
   - Mittel (L2, L3) zur optischen Konjugation, die auf die Messachse zentriert sind und mit denen die optische Konjugation zwischen der vorbestimmten Ebene des Auges, der Ebene der Lichtblende und der Analyseebene gewährleistet werden kann,

   **dadurch gekennzeichnet, dass** der Lichtstrahl in der Mitte des Filterelements (FLT) konvergiert, dass das Filterelement, der Lichtweg, der Analyseweg, die Trennmittel und die Konjugationsmittel fest verbunden und auf einer Platte (PTF1) positioniert sind, die gegenüber den Abbildungsmitteln (L1) entlang der optischen Achse der Mittel (z) beweglich ist, wodurch über die Abbildungsmittel (L1) die Einstellung der optischen Konjugation der Netzhaut des Auges mit der Mitte des Filterelements in Abhängigkeit von der Ametropie des Auges möglich ist, und dass die Lichtblende bezüglich der Messachse bzw. bezüglich jeglicher zur Messachse optisch konjugierten Achse dezentriert ist, derart, dass der von der Hornhaut des Auges, der Abbildungsmittel (L1) und jeglichem Diopter, das sich zwischen dem Filterelement und der Hornhaut befindet, reflektierte Störlichtstrom, über das Filterelement (FLT) aus dem Analyseweg umgeleitet wird.

2. Vorrichtung nach Anspruch 1, **dadurch gekennzeichnet, dass** das Filterelement (FLT) aus einem lichtdichten Lochschild gebildet ist, wobei das Loch auf die Messachse (z) zentriert ist und vorbestimmte Abmessungen aufweist, derart, dass der Lichtstrahl (FE) und der Analysestrahl (FA), die im wesentlichen auf den Mittelpunkt des Lochs fokussiert sind, von dem Filterelement übertragen werden, während die Störreflexionen von dem lichtdichten Teil des Filterelements abgeschirmt werden.

3. Vorrichtung nach Anspruch 1, **dadurch gekennzeichnet, dass** das Filterelement (FLT) virtuell ist und durch die Abbildung eines reellen Filterelements (FLTO) gebildet wird, wobei das reelle Filterelement (FLTO) auf dem Analyseweg VA angeordnet ist.

4. Vorrichtung nach Anspruch 3, **dadurch gekennzeichnet, dass** das reelle Filterelement (FLTO) aus einem lichtdichten Lochschild gebildet ist.

5. Vorrichtung nach den Ansprüchen 2 oder 4, **dadurch gekennzeichnet, dass** die Größe des Lochs des lichtdichten Schildes kontrollierbar ist.

6. Vorrichtung nach Anspruch 1, **dadurch gekennzeichnet, dass** das Filterelement (FLT) aus einem Lochspiegel gebildet ist, wobei das Loch auf die Messachse (z) zentriert ist und vorbestimmte Abmessungen aufweist, derart, dass der Lichtstrahl (FE) und der Analysestrahl (FA), die im wesentlichen auf den Mittelpunkt des Lochs fokussiert sind, von dem Filterelement übertragen werden, während die Störreflexionen über den reflektierenden Teil des Filterelements abgelenkt werden.

7. Vorrichtung nach Anspruch 1, **dadurch gekennzeichnet, dass** das Filterelement (FLT) einen Spiegel aufweist, der auf die Messachse zentriert ist und vorbestimmte Abmessungen aufweist, derart, dass der Lichtstrahl (FE) und der Analysestrahl (FA), die im wesentlichen auf den Mittelpunkt des Spiegels fokussiert sind, von dem Filterelement reflektiert werden, während die Störreflexionen nicht über das Filterelement abgelenkt werden.

8. Vorrichtung nach einem der vorangehenden Ansprüche, **dadurch gekennzeichnet, dass** sie ferner Steuermittel (COM) zum Steuern der Platte (PTF1) aufweist, die mit den Analysemitteln verbunden sind und eine Verlagerung der Platte entlang der Messachse (z) gemäß einer vorbestimmten Funktion der von den Analysemitteln gemessenen Fehlsichtigkeit des Auges bewirken.

9. Vorrichtung nach einem der vorangehenden Ansprüche, **dadurch gekennzeichnet, dass** die Mittel zum Positionieren des Auges insbesondere einen Abbildungsweg (VI) für die Pupille des Auges (P0) mit einem im wesentlichen auf die Messachse (z) zentrierten Objektiv (L4) und einer fest mit der Platte (PTF1) verbundenen Abbildungskamera mit einem in einer Fokalebene des Objektivs (L4) befindlichen Sensor und einem Anzeigesystem aufweisen.

10. Vorrichtung nach Anspruch 9, **dadurch gekennzeichnet, dass** die Positioniermittel ferner eine Leuchtvorrichtung zum Erleuchten des Auges aufweisen, die aus einer Einheit von Lichtquellen gebildet ist, wobei die Positionierung unter Kontrolle der Scharfstellung der Abbildung der von der Hornhaut auf den Sensor reflektierten Quellen angepasst wird, wodurch eine Kontrolle der Positionierung der den Scheitelpunkt der Hornhaut des Auges berührenden Ebene möglich ist.

11. Vorrichtung nach einem der Ansprüche 9 oder 10, **dadurch gekennzeichnet, dass** die Positioniermittel ferner eine quasipunktförmige Lichtquelle aufweisen, die auf die Messachse (z) zentriert und im wesentlichen im Brennpunkt des Objektivs (L4) positioniert ist, sowie eine Maske, die aus zumindest zwei Öffnungen gebildet ist, von denen zumindest die eine gegenüber der Messachse dezentriert ist, und die in der Nähe der genannten Quelle positioniert ist, um zumindest zwei Lichtstrahlen zu bilden, wobei ein Strahlenteiler die Strahlen empfängt und die Positionierung angepasst wird, indem am Sensor des Abbildungsweges die Übereinanderlagerung der Flecke kontrolliert wird, die von den Strahlen nach Reflexion an der Hornhaut des Auges gebildet werden, wodurch somit eine Kontrolle der Positionierung der den Scheitelpunkt der Hornhaut des Auges berührenden Ebene möglich ist.

12. Vorrichtung nach einem der Ansprüche 9 bis 11, **dadurch gekennzeichnet, dass** die Mittel zum Positionieren des Auges ferner an der Anzeige des Sensors des Abbildungsweges Mittel zum Erfassen der lateralen Stellung der Pupille des Auges aufweisen, mit denen es möglich ist, die genannte Stellung gegenüber der Stellung des Lichtstrahls in Abhängigkeit von der Größe der Pupille anzupassen.

13. Vorrichtung nach einem der Ansprüche 9 bis 12, **dadurch gekennzeichnet, dass** die Positioniermittel ferner Mittel zum gemeinsamen Verlagern der Abbildungsmittel (L1) und der Platte (PTF1) aufweisen, um das Anpassen der Positionierung des Auges vorzunehmen.

14. Vorrichtung nach einem der vorangehenden Ansprüche, **dadurch gekennzeichnet, dass** die Mittel zur optischen Konjugation Mittel (L2) zum Abbilden auf dem Analyseweg aufweisen, wobei die Analyseebene mit einer Fokalebene der Abbildungsmittel zusammenfällt, sowie Mittel (L3) zum Abbilden auf dem Lichtweg, wobei die Ebene der Lichtblende mit einer Fokalebene der genannten Abbildungsmittel zusammenfällt, wobei die genannten Mittel (L3) auch die Fokussierung des Lichtstrahls auf die Mitte des Filterelements (FLT) gewährleisten.

15. Vorrichtung nach einem der vorangehenden Ansprüche, **dadurch gekennzeichnet, dass** die Trennmittel zumindest einen ersten Strahlungsteiler (LM1) mit im wesentlichen ebenen und parallel verlaufenden Flächen aufweisen, der zur Messachse (Z) geneigt verläuft, von einem Teil des Lichtstrahls durchbrochen wird und einen Teil der von dem Auge austretenden Welle zum Analyseweg hin reflektiert.

16. Vorrichtung nach Anspruch 15, **dadurch gekennzeichnet, dass** der Reflexionskoeffizient des Strahlungsteilers (LM1) über etwa 70 % liegt.

17. Vorrichtung nach einem der Ansprüche 15 oder 16, **dadurch gekennzeichnet, dass** durch die Dicke des Strahlungsteilers (LM1) ein vorbestimmter Versatz bezüglich der Messachse (z) in den Lichtstrahl eingebracht wird, derart, dass bei einem versehentlichen Fehlen des Strahlungsteilers der Lichtstrahl nicht mehr durch das Filterelement tritt.

18. Vorrichtung nach einem der vorangehenden Ansprüche, **dadurch gekennzeichnet, dass** sie ferner einen Fixierweg aufweist, mit dem die Blickrichtung des Patienten, dessen Auge analysiert wird, fixiert werden kann, sowie eine beleuchtete Abbildung und ein Objektiv (L5), wobei das Objektiv im wesentlichen die optische Konjugation der Abbildung mit dem Filterelement (FLT) gewährleistet.

19. Vorrichtung nach einem der vorangehenden Ansprüche, **dadurch gekennzeichnet, dass** die Analysemittel einen Wellenfrontanalysator aufweisen, der als Shack-Hartmann-Sensor ausgeführt ist und insbesondere eine Mikrolinsenmatrix, einen Matrixsensor und Verarbeitungsmittel aufweist, wobei die Analyseebene der Ebene der Mikrolinsenmatrix entspricht.

20. Vorrichtung nach einem der vorangehenden Ansprüche, **dadurch gekennzeichnet, dass** der Lichtweg ferner Mittel zum Aussenden eines auf die Messachse (z) zentrierten Lichtstrahls mit vorbestimmtem Durchmesser aufweist, der in der Mitte des Filterelements (FLT) konvergiert, und dass die Stellung der Platte (PTF1) entlang der Messachse (z) angepasst wird, um im wesentlichen die optische Konjugation des Krümmungsmittelpunktes der Hornhaut mit der Mitte des Filterelements (FLT) durch die Abbildungsmittel (L1) zu gewährleisten, wodurch die Autokollimation des Lichtstrahls auf der Vorderseite der Hornhaut möglich ist, wobei der an der genannten Seite reflektierte Lichtstrom den Analysemitteln (MA) zugeführt wird, um die Messung der Topographie der Hornhaut vorzunehmen.

21. Vorrichtung nach einem der Ansprüche 1 bis 19, **dadurch gekennzeichnet, dass** der Lichtweg ferner Mittel zum Aussenden eines auf die Messachse (z) zentrierten Lichtstrahls mit vorbestimmtem Durchmesser aufweist, der in der Mitte des Filterelements (FLT) konvergiert, und dass die Abbildungsmittel ein zusätzliches und abnehmbares Autokollimationssystem aufweisen, mit dem der Lichtstrahl (FE) im Krümmungsmittelpunkt der Hornhaut fokussiert werden kann, wobei der an der Vorderseite der Hornhaut reflektierte Lichtstrom den Analysemitteln (MA) zugeführt wird, um die Messung der Topographie der Hornhaut vorzunehmen.

Figure 1A

Figure 1B

Figure 2

EYE

FE
FA

ECL

PTF2

ECL

LM1

FLT

LM2

L3

L4

FE

DET

L2

PE

APT

IMA

PA

SRC

MA

COM

SCR

TRT

PTF1

Figure 3

Figure 4

Figure 5

Figure 6

Figure 7

EP 1 427 329 B1

Figure 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

•  US 0577719 A, Williams

**Littérature non-brevet citée dans la description**

•  **LIANG et al.** Objective measurement of wave aberrations of the human eye with the use of a Schack-Hartman wave-front sensor. *J.Opt.Soc.Am.A,* 1994, vol. 11 (7), 1-9

•  wave-front estimation from wave-front slope measurements. *J. Opt. Soc. Am.,* Août 1980, vol. 70 (8